# EUROPEAN PATENT APPLICATION

(11) **EP 0 928 832 A2**
(43) Date of publication of application: **14.07.1999**
(21) Application number: 99300170.0
(22) Date of filing: 11.01.1999
(51) Int. Cl.: C12Q 1/68

(54) **Cystic fibrosis test based on the detection of mutations in the CFRE gene by ARMS**

(30) Priority: 13.01.1998 GB 9800536
(71) Applicant: ZENECA LIMITED, London W1Y 6LN (GB)
(72) Inventor: Weston, Susan Louise, Northwich, Cheshire CW9 7RA (GB); Kelly, Stephen James, Northwich, Cheshire CW9 7RA (GB); Robertson, Nancy Hastings, Northwich, Cheshire CW9 7RA (GB)
(74) Representative: Phillips, Neil Godfrey Alasdair

(57) **Abstract**

A method for detecting the presence or absence of twelve mutations in the cystic fibrosis transmembrane conductor regulator (CFTR) gene, which method comprises contacting sample genomic DNA from an individual in two separate reaction vessels with allele specific primer sets for (A) 1717-1 G>A, G542X, W1282X, N1303K, AF508(M), 3849+10kb C>T mutations and (B) the 621+1 G>T, R553X, G551D, R117H, R1162X and R334W mutations respectively, in the presence of appropriate nucleotide triphosphates and an agent for polymerisation, such that each diagnostic primer is extended only when the relevant mutation is present in the sample; and detecting the presence or absence of CFTR gene alleles by reference to the presence or absence of diagnostic primer extension product(s).

### Annex to the description

SEQUENCE LISTING

(1) GENERAL INFORMATION:

(i) APPLICANT:
(A) NAME: Zeneca Ltd
(B) STREET: 15 Stanhope Gate
(C) CITY: London
(D) STATE: Greater London
(E) COUNTRY: England
(F) POSTAL CODE (ZIP): W1Y 6LN
(G) TELEPHONE: 0171 304 5000
(H) TELEFAX: 0171 304 5151
(I) TELEX: 0171 834 2042

(ii) TITLE OF INVENTION: SEQUENCES

(iii) NUMBER OF SEQUENCES: 27

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(vi) PRIOR APPLICATION DATA:
(A) APPLICATION NUMBER: GB 9800536.6
(B) FILING DATE: 13-JAN-1998

(2) INFORMATION FOR SEQ ID NO: 1:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 1:

GAGCACAGTA CGAAAAACCA CCT 23

(2) INFORMATION FOR SEQ ID NO: 2:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 2:

AAACTTTTAC AGGGATGGAG AACG 24

(2) INFORMATION FOR SEQ ID NO: 3:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 3:

AGAGGATTAT CTATGCAAAT CCTTGTAACC 30

(2) INFORMATION FOR SEQ ID NO: 4:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 4:

TCAACTTCAC TATCAAAAGT CATCATCTAG 30

(2) INFORMATION FOR SEQ ID NO: 5:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 5:

TCTTGGGATT CAATAACTTT GCAACAGTCA 30

(2) INFORMATION FOR SEQ ID NO: 6:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 6:

GAATTCCCAA ACTTTTAGAG ACATC 25

(2) INFORMATION FOR SEQ ID NO: 7:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 7:

TACTAAAAGT GACTCTCTAA TTTTCTATTT TTGGTAATTA 40

(2) INFORMATION FOR SEQ ID NO: 8:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 8:

AGTTTGCAGA GAAAGACAAT ATAGTTCTCT 30

(2) INFORMATION FOR SEQ ID NO: 9:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 9:

TAATCTCTAC CAAATCTGGA TACTATACC 29

(2) INFORMATION FOR SEQ ID NO: 10:

(i) SEQUENCE CHARACTERISTICS:
(A) LENGTH: 30 base pairs

(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 10:

TGATCACTCC ACTGTTCATA GGGATCCATC 30

(2) INFORMATION FOR SEQ ID NO: 11:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 11:

AATTTGAGAG AACTTGATGG TAAGTACA 28

(2) INFORMATION FOR SEQ ID NO: 12:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 12:

GTATCTATAT TCATCATAGG AAACACCATT 30

(2) INFORMATION FOR SEQ ID NO: 13:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 13:

CCAGACTTCA CTTCTAATGA TGATTATGGG 30

(2) INFORMATION FOR SEQ ID NO: 14:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 14:

ACATTTCCTT TCAGGGTGTC TGACTAA 27

(2) INFORMATION FOR SEQ ID NO: 15:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 15:

TTGTGGATCA AATTTCAGTT GACTTGTCAT C 31

(2) INFORMATION FOR SEQ ID NO: 16:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 16:

GTATCTATAT TCATCATAGG AAACACCACA 30

(2) INFORMATION FOR SEQ ID NO: 17:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 17:
   GACTTCACTT CTAATGATGA TTATGGGAGA 30

(2) INFORMATION FOR SEQ ID NO: 18:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 18:

TGCCATGGGG CCTGTGCAAG GAAGTATTGA 30

(2) INFORMATION FOR SEQ ID NO: 19:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 19:

AGCCTATGCC TAGATAAATC GCGATAGACT 30

(2) INFORMATION FOR SEQ ID NO: 20:

(i) SEQUENCE CHARACTERISTICS:
(A) LENGTH: 37 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 20:

GTTTCACATA GTGTATGACC CTCTATATAC ACTCATT 37

(2) INFORMATION FOR SEQ ID NO: 21:

(i) SEQUENCE CHARACTERISTICS:
(A) LENGTH: 30 base pairs
(B) TYPE: nucleic acid

(C) STRANDEDNESS: single
(D) TOPOLOGY: linear

(ii) MOLECULE TYPE: other nucleic acid

(xi) SEQUENCE DESCRIPTION: SEQ ID NO: 21:

CCTATGCACT AATCAAAGGA ATCATCCTGT 30

(2) INFORMATION FOR SEQ ID NO: 22:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 22:

TTTGTTTATT GCTCCAAGAG AGTCATACCA 30

(2) INFORMATION FOR SEQ ID NO: 23:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 23:

GCTAAAGAAA TTCTTGCTCG TTGTT 25

(2) INFORMATION FOR SEQ ID NO: 24:

(i) SEQUENCE CHARACTERISTICS:
(A) LENGTH: 60 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 24:

GACTGACTGA CTGACTGACT CTGACTGACT TATTCACCTT GCTAAAGAAA TTCTTGCTGA 60

(2) INFORMATION FOR SEQ ID NO: 25:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 25:

TAAAATTGGA GCAATGTTGT TTTTGACC 28

(2) INFORMATION FOR SEQ ID NO: 26:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 26:

TATTTTTATT TCAGATGCGA TCTGTGAGTT 30

(2) INFORMATION FOR SEQ ID NO: 27:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO: 27:

TTTTGCTGTG AGATCTTTGA CAGTCATTT 29

## Description

Cystic Fibrosis (CF) is the most common fatal autosomal recessive disease affecting Caucasian populations. CF has an incidence of 1 in 2 000 to 3 000 births depending on population group¹ and this indicates a carrier frequency of around 1 in 25 (i.e. 4% of the population). The prognosis for an affected child with CF is a median life expectancy currently estimated to be 40 years². Since the discovery of the CFTR gene³ over 600 CF associated mutations have been identified. The majority of these are presumed to be disease-causing but most are rare so many laboratories test for a limited number with an emphasis on those predominating in their local populations.

Detection methods used to date have included DNA sequencing, DNA enzyme immunoassay (Sanguiolo et al, Int.J.Clin.Lab.Res., 1995, 25, 142-145), multiplex DGGE analysis (Costes et al, Hum.Mutat., 1993, 2, 185-191), and the use of the polymerase chain reaction (PCR) in conjunction with allele-specific oligonucleotide probes (PCR-ASO).

In addition, ARMS is an established technique that enhances the utility of the PCR for the detection of mutations and other polymorphisms in DNA⁴ (see our European Patent No. 0 332 435). ARMS also provides the means to distinguish between homozygotes and heterozygotes for any given allelic variation. The principle of the method is that under appropriate conditions an oligonucleotide which is not matched to target (genomic) DNA sequence at its 3'-end will not be extended by *Taq* DNA polymerase. The 3'-end of an ARMS primer therefore confers its allele-specificity. Hence, an ARMS product is only generated if the primer is complementary to its target at the 3'-end under the appropriate conditions. We⁵ and others^{6,7} have described the use of ARMS to test for ÆF508, the most common mutation of the CFTR gene³. We have also shown that four ARMS analyses can be performed simultaneously by multiplexing allele-specific primers⁸ and our observations have been confirmed by Fortina *et al*⁹.

However the need still exists for further CF tests, in particular for rapid and reliable methods for the extensive investigations routinely performed in many laboratories. We have now devised and validated a two-tube multiplex ARMS test, hereinafter referred to as the CF(12)m test, which detects 12 of the most prevalent CF mutations¹⁰ simultaneously. The CFTR gene mutations that are detected by the test are 1717-1G>A, G542X, W1282X, N1303K, ΔF508, 3849+10kb C>T, 621+1 G>T, R553X, G551D, R117H, R1162X and R334W which are described in reference 10 and papers cited therein. The test also distinguishes between CF AF508 heterozygotes and homozygotes. The CF Genetic Analysis Consortium data¹⁰ allows the minimum detection capability of the test to be calculated by country, as shown in Table 1. Similarly calculated continental values are shown in Table 2. A further observation from these data is the very high minimum detection capability for Ashkenazic Jews which is calculated to be 95.4%.

Therefore in a first aspect of the present invention we provide a method for detecting the presence or absence of twelve mutations in the cystic fibrosis transmembrane conductor regulator (CFTR) gene, which method comprises contacting sample genomic DNA from an individual in two separate reaction vessels with allele specific primer sets for (A) 1717-1 G>A, G542X, W1282X, N1303K, ΔF508(M), 3849+10kb C>T mutations and (B) the 621+1 G>T, R553X, G551D, R117H, R1162X and R334W mutations respectively, in the presence of appropriate nucleotide triphosphates and an agent for polymerisation, such that each diagnostic primer is extended only when the relevant mutation is present in the sample; and detecting the presence or absence of CFTR gene alleles by reference to the presence or absence of diagnostic primer extension product(s).

Allele specific primer sets A and B as defined above represent further independent aspects of the invention.

Advantageously, primer set B also includes an allele specific primer for the normal ΔF508 allele (ΔF508(N)), ie. DNA sequence unaffected by the ΔF508 mutation.

The test sample of nucleic acid is conveniently extracted from a sample of blood, mouthwash or other body fluid from an individual. Also buccal-swab samples may be used. It will be appreciated that the test sample may equally be a nucleic acid sequence corresponding to the sequence in the test sample. That is to say that all or a part of the region in the sample nucleic acid may firstly be amplified using any convenient technique such as PCR before use in the method of the invention. The methods of the invention are conveniently effected using about 20-100 nanograms of template DNA.

Any convenient enzyme for polymerisation may be used provided that it does not affect the ability of the DNA polymerase to discriminate between normal and mutant template sequences to any significant extent. Examples of convenient enzymes include thermostable enzymes which have no significant 3'-5' exonuclease activity, for example *Taq* DNA polymerase, particularly "Ampli Taq Gold"™ DNA polymerase (PE Applied Biosystems), Stoffel fragment, or other appropriately N-terminal deleted modifications of *Taq* or *Tth* (*Thermus thermophilus*) DNA polymerases.

We have now devised allele specific primers for the above CFTR gene mutations which have been shown, in primer sets A and B, to detect the specific mutations reliably and robustly. Therefore in further aspects of the invention we provide individual diagnostic primers and sets of diagnostic primers A and B as disclosed below:

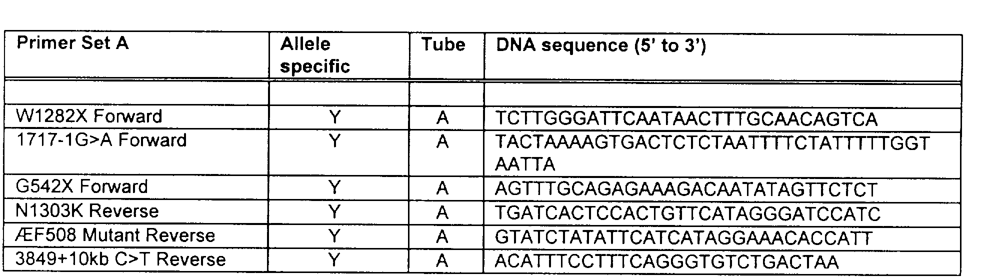

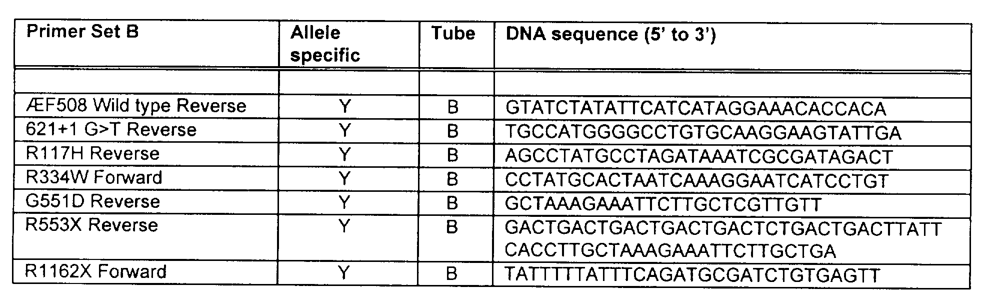

Although the above primers have been optimised for use in the diagnostic methods of the invention, it will be appreciated that some modification of some or all of the above primers may be possible without adversely affecting their performance in the methods of the invention. Such modifications may be determined by the molecular biologist of ordinary skill in comparative experiments. Whilst we do not wish to be bound by theoretical considerations, in general, one or more of the nucleotides at the 5' end of a primer, may be substituted for other (non-complementary) nucleotides, for example about 1 in 4, 1 in 5, lin 6, 1 in 7, such as 1 in 8, 1 in 9 or 1 in 10 of the nucleotides may be substituted. Nearer to the 3' end of the primer the scope for modification is more limited, within about six bases of the 3' end, one or possibly two nucleotides may be substituted for other (non- complementary) nucleotides. The 3' terminal nucleotide of the primer should not be modified.

Additions to and deletions from the above primer sequences are also possible but not preferred. Again a molecular biologist may perform comparative experiments to reveal what modifications are practicable within the scope of the present invention.

The primers may be manufactured using any convenient method of synthesis. Examples of such methods may be found in standard textbooks, for example *"Protocols For Oligonucleotides And Analogues: Synthesis And Properties;"* Methods In Molecular Biology Series; Volume 20; Ed. Sudhir Agrawal, Humana ISBN: 0-89603-247-7; 1993; 1^{st} Edition.

In many situations, it will be convenient to use the sets of diagnostics primer of the invention in combination with further amplification primers in one or more cycles of PCR amplification. A convenient example of this aspect is set out in our European patent number EP-B1-0332435. The further (amplification) primer is either a forward or a reverse common primer. Hot-start PCR is conveniently employed.

Preferred individual primers and sets of further primers are set out below.

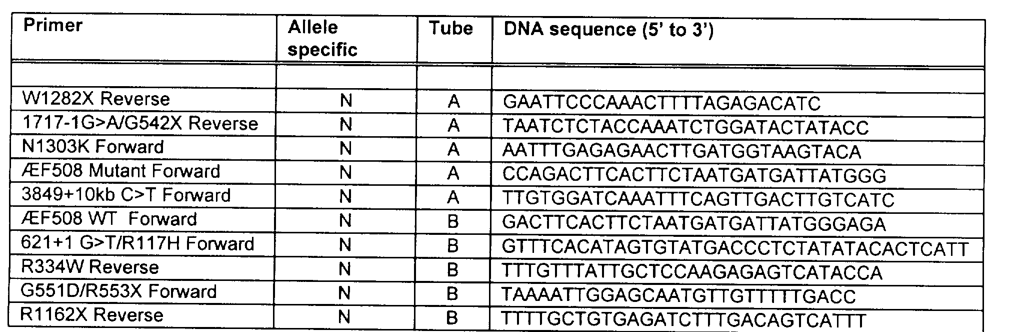

In a preferred aspect of the invention primer mixes A and B are provided which mixes A and B comprise both allele specific and amplification primers as follows:

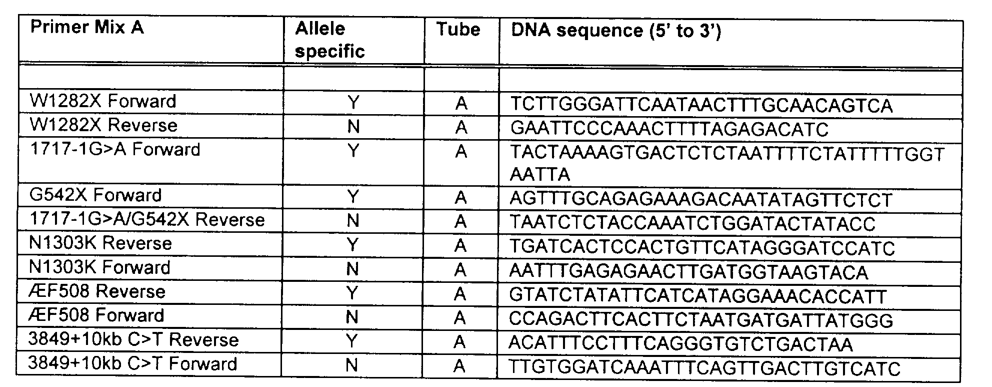

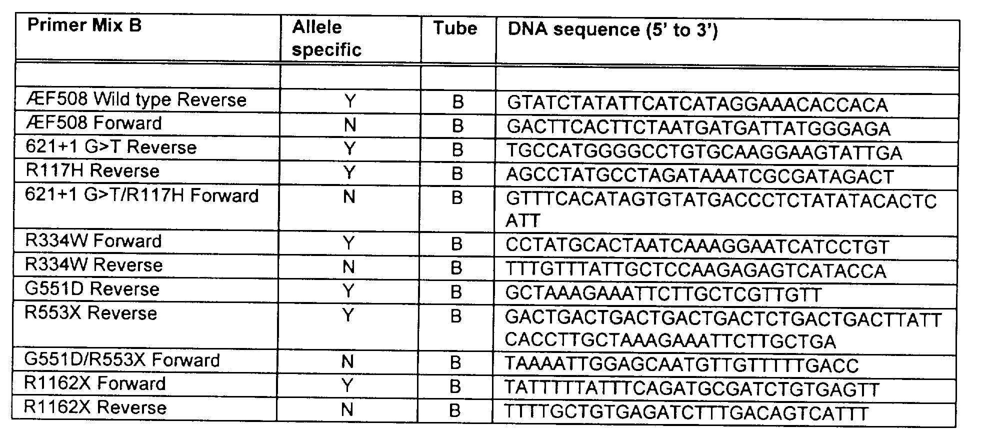

Any convenient control primer(s) may be used to confirm correct functioning of the PCR.. Two independent control reactions may be provided with each primer set. For example, one amplifies a region of the apolipoprotein (Apo B) gene, the other amplifies a region of the ornithine decarboxylase (ODC) gene. The Apo B reaction is designed to amplify less efficiently than the ARMS reactions ensuring that this control reaction will fail preferentially if PCR variables are not within the defined tolerances of the test. The Apo B control therefore safeguards against a false negative result if the test is performed under sub-optimal conditions. Both control product bands are preferably present for a reliable diagnosis to be made.

Preferred control primers include one or more of the following:

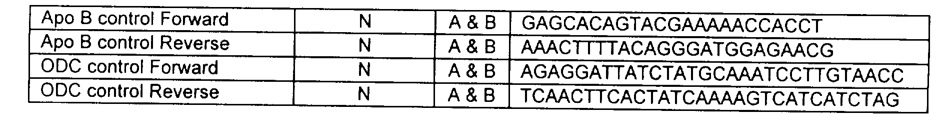

It will be appreciated that any of the primers set out hereinbefore may be modified as outlined in respect of primer sets A and B.

A variety of methods may be used to detect the presence or absence of diagnostic primer extension products and/or amplification products. These will be apparent to the person skilled in the art of nucleic acid detection procedures. Preferred methods avoid the need for radiolabelled reagents. Particular detection methods include those which provide size or sequence differentiation, preferably gel electrophoresis based methods. For example, primer extension products are separated by agarose gel electrophoresis and visualised by ethidium bromide staining/uv transillumination. Individual mutations are conveniently identified by size comparison of the products against an appropriate DNA ladder marker. Alternative detection methods include capillary zone electrophoresis (CZE).

One or more of the diagnostic primer sets/mixes of the invention may be conveniently packaged with instructions for use in the method of the invention and appropriate packaging and sold as a kit. The kits will conveniently include one or more of the following: corresponding amplification primers, appropriate nucleotide triphosphates, for example dATP, dCTP, dGTP, dTTP, a suitable polymerase as previously described, and optimised buffer solution.

A preferred kit of the invention comprises primer mixes A and B (as defined above) in separate containers, taq DNA polymerase (preferably Amplitaq Gold), and optimised enzyme dilution buffer.

The invention will now be illustrated but not limited by reference to the following detailed description, References, Examples, Tables and Figures wherein
Table 1 shows the minimum detection capabilities of the CF(12)m ARMS test; average figures for countries testing >100 chromosomes¹⁰.
Table 2 shows the minimum detection capabilities of the CF(12)m ARMS test; analysis by continent derived from data in reference 10. Empty cells indicate that not all centres screened for each mutation detected by the test; therefore the actual detection capabilities for some mutations may be higher.
Table 3 shows the analysis of the 754 chromosomes tested. "Confirmatory typing as detailed in references cited within reference 10; ASO, Allele-specific oligonucleotide hybridisation.
Table 4 shows primer sequences used in the CF(12)m ARMS test.

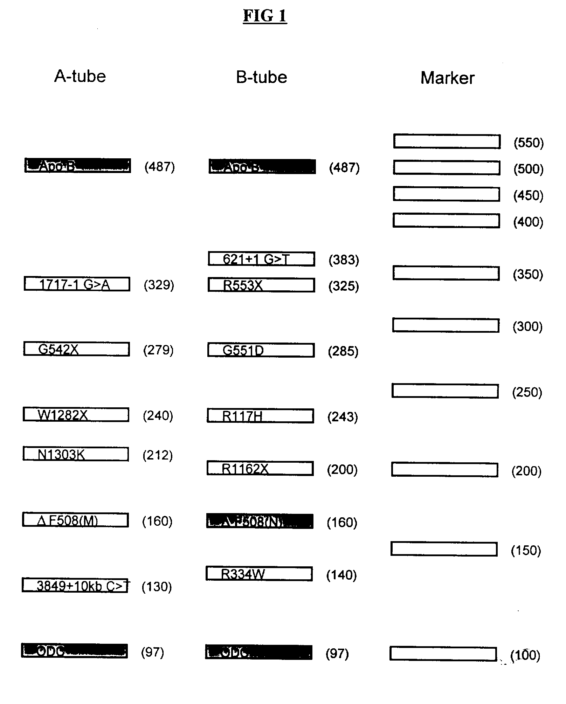

Figure 1 shows a diagramatic representation of electrophoretic mobilities of ARMS, control and size marker gel bands. The respective ARMS and control bands are labelled, all band sizes (bp) are shown in parentheses. The 250 bp marker band (hatched) is more intense to provide a reference position on the gels. In samples which do not contain any of the mutations detected by the test, only the Apo B and ODC control bands (both tubes) and ΔF508 normal band (B-tube) will be visible, these are shown shaded.

### References:

1. Welsh MJ, Tsui L-C, Boat TF, In Scriver CR, Beaudet AL. Sly WL, Valle (eds), The metabolic and molecular basis of inherited disease. McGraw-Hill, New York: 3799-3876, 1990.
2. Elborn JS, Shale DJ, Britton JR. Cystic fibrosis current survival and population estimates to the year 2000.*Thorax* 1991;46:881-885.
3. Riordan JR, Rommens JM, Kerem B, Alon N, Rozmahel R, Grzelczak Z, *et al.* Identification of the cystic fibrosis gene: cloning and characterization of complementary DNA. *Science* 1989;**245**:1066-1073.
4. Newton CR, Graham A, Heptinstall LE, Powell SJ, Summers C, Kalsheker N, *et al*. Analysis of any point mutation in DNA. The amplification refractory mutation system (ARMS). *Nucl Acids Res* 1989;**17**:2503-2516.
5. Newton CR, Heptinstall LE, Summers C, Super M, Schwarz M, Graham A, *et al*. Detection of delta-F508 deletion by amplification refractory mutation system. *Lancet* 1990;**i**:1217-1219.
6. Ballabio A, Gibbs RA, Caskey CT. PCR test for cystic fibrosis deletion. *Nature* 1990;**343**:220.
7. Wagner M, Schloesser M, Reiss J. Direct gene diagnosis of cystic fibrosis by allele-specific polymerase chain reactions. *Mol Biol Med* 1990;**7**:359-364.
8. Ferrie RM, Schwarz MJ, Robertson NH, Vaudin S, Super M, Malone G, *et al.* Development, multiplexing and application of ARMS tests for common mutations in the CFTR gene. *Am J Hum Genet* 1992;**51**:251-62.
9. Fortina P, Conant R, Monokian G, Dotti G, Parrella T, Hitchcock W, *et al.* Non-radioactive detection of the most common mutations in the cystic fibrosis transmembrane conductance regulator gene by multiplex allele-specific polymerase chain reaction. *Hum Genet* 1992;**90**:375-378.
10. Kazazian HH. Population variation of common cystic fibrosis mutations. *Hum Mutat* 1994;**4**:167-177.
11. Kwok S, Higuchi R. Avoiding false positives with PCR. *Nature* 1989;**339**:237-238.
12. Super M, Schwarz MJ, Malone G, Roberts T, Haworth A, Dermody G. Active cascade testing for carriers of cystic fibrosis gene. *BMJ* 1994;**308**:1462-1467.
13. Wald NJ. Couple screening for cystic fibrosis. *Lancet* 1991;**338**:1318-1319.
14. Gilfillan A, Axton R, Brock DJH. Mass screening for cystic fibrosis heterozygotes: Two assay systems compared. *Clin Chem* 1994;**40**:197-199.
15. Livingstone J, Axton RA, Gilfillan A, Mennie M, Compton M, Liston WA, *et al.* Antenatal screening for cystic fibrosis: a trial of the couple model. *BMJ,* 1994; **308**:1459-1462.
16. Findlay I, Cuckle H, Lilford RJ, Rutherford RJ, Quirke P, Lui S. Screening sperm donors for cystic fibrosis. *BMJ* 1995;**310**:1533.
17. Cuppens H, Cassiman JJ. A quality control study of CFTR mutation screening in 40 different European laboratories: The European concerted action on cystic fibrosis. *Eur J Hum Genet* 1995;**3**:235-245.

### Example 1

For clinical applications it is essential that the results obtained using PCR based tests are both reliable and reproducible. It is generally understood that the efficiency of PCR can be affected by a number of variables and it is important to understand which ones might affect test performance. We have therefore investigated the effects of changes of primer, DNA template and *Taq* DNA polymerase concentrations and of PCR annealing temperatures. These studies have defined the conditions under which neither false positive nor false negative results are produced even if the test is performed under sub-optimal conditions. We have unequivocally typed DNA samples that were obtained from different laboratories and that were prepared by a variety of methods.

### Methods

### DNA samples

The panel of samples was selected to include normal DNAs, DNA from CF ΔF508 homozygotes and several examples of each mutation for which the test was designed to detect. All DNA samples were prepared from EDTA/blood. DNA samples from external sources were prepared and typed independently using standard recognised procedures and typed by the methods outlined in table 3. DNA samples from CF unaffected individuals were prepared as described previously⁸.

### Primer design

In designing the ARMS primers it was important to ensure that a false result could not arise due to other DNA sequence variations at the same site or in the vicinity of the mutations tested for. For example, ΔF508 and non-ΔF508 alleles should not be confused for either the mutant ΔI507, or the benign F508C alleles. This discrimination was achieved after particular consideration and the appropriate choice of orientation of each primer with respect to the direction of transcription of the CFTR gene. Careful attention was also given to the inclusion of additional base-pair mismatches between each primer and the genomic DNA sequence and also the length and concentration of each primer. In combining primers for multiplex analysis, many primers are included in one reaction mix. It was therefore necessary to minimise any primer interactions that might affect the test performance. The ARMS primer sequences are shown in Table 4.

### Test design

The CF(12)m test consists of two tubes, the A and the B tube. The A tube contains ARMS primers specific for the 1717-1G>A, G542X, W1282X, N1303K, AF508, and 3849+10kb C>T mutations. The B tube contains ARMS primers specific for the 621+1 G>T, R553X, G551D, R117H, R1162X and R334W mutations. The B tube also contains an ARMS primer specific for the normal AF508 allele. There are also two control reactions in each tube. One amplifies a region of the apolipoprotein (Apo B) gene, the other amplifies a region of the ornithine decarboxylase (ODC) gene. The Apo B reaction is designed to amplify less efficiently than the ARMS reactions ensuring that this control reaction will fail preferentially if PCR variables are not within the defined tolerances of the test. The Apo B control therefore safeguards against a false negative result if the test is performed under sub-optimal conditions. Both control product bands should be present for a reliable diagnosis to be made. The concentrations of the component ARMS primers in each tube were adjusted to amplify their respective targets with equal efficiency. The primer DNA sequences are shown in Table 4. The remaining constituents of each tube comprise ARMS buffer; 10mM Tris-HCl, (pH 8.3) 1.2mM MgCl₂, 50mM KCl, 0.01% gelatin, dNTPs (100µM each). Genomic DNA and *Taq* or *Amplitaq Gold* DNA polymerase are added at the start of the test.

The CF(12)m test output is shown (Figure 1)

### Test method

The test is performed by adding genomic DNA prepared from EDTA/blood to each tube which is then heated at 94°C, 5 minutes. *Taq* DNA polymerase (2.5 units) is added to each tube, whilst maintaining the temperature at 94°C, and thermal cycling (35 cycles of 94°C, 1 minute; 58°C, 2 minutes; 72°C, 1 minute with a final extension at 72°C, 10 minutes) is initiated. Alternatively, both *Amplitaq Gold* DNA polymerase (2.5 units) and genomic DNA prepared from EDTA/blood are added to each tube and thermal cycling (as described above) initiated following a 94°C, 20 minutes incubation during which the DNA polymerase is activated.

The amplification products are separated by agarose gel electrophoresis against a 50 base-pair DNA ladder marker (Pharmacia Biotech) and visualised by UV transillumination. All procedures generally accepted for avoiding PCR carry-over contamination¹¹ were employed during the development and validation of the test and are recommended when using the test. The presence of the control products and a specific ARMS product, defined by electrophoretic mobility, is diagnostic of the presence of the respective mutant allele and/or normal ΔF508 allele (Figure 1).

### Results

Test performance:

A series of experiments was performed to determine the tolerance of the CF(12)m ARMS test system to the variation of primer concentrations, annealing temperatures, *Taq* or *Amplitaq Gold* DNA polymerase concentration, the input DNA amount and the method of its extraction. This served to demonstrate the permissible limits of each test variable and to confirm that a 'non-result' or test fail, rather than an incorrect result was observed should any variable fall outside defined tolerances. The CF(12)m test was functional at +/-2 °C of the standard annealing temperature (58 °C) Under sub-optimal conditions (3 °C above the standard temperature) all diagnostic product bands were visible but the upper control product band was absent. This feature of the test prevents the occurrence of false negative results due to operation at higher temperatures, since both control product bands must be present to make a diagnosis. The results demonstrate that the test remains functional over a broad range of annealing temperatures. The effects of the primer, *Taq* or *Amplitaq Gold* DNA polymerase and DNA concentrations were assessed in an analogous manner. This defined the respective windows within which both of the control bands and each mutation-specific band was appropriately generated.

383 DNA samples prepared from EDTA/blood were analysed using the CF(12)m test. and 377 gave results in agreement with those obtained independently. Six samples (1.57%) were not scored due to the absence of one or both control bands. These were all archival samples known to be of low concentration and degradation of these samples could not be eliminated. Where rare non-ΔF508 compound heterozygotes have been obtained (3849+10kb C>T/W1282X; 3849+10kb C>T/G542X; G542X/N1303K; G542X/W1282X; G551D/R553X; N1303K/1717-1G>A; G542X/1717-1G>A; N1303K/W1282X; R553X/R334W) and analysed, both mutations were correctly identified. Similarly, all ΔF508 compound heterozygotes were correctly typed. We also observed that there was no mistyping when ΔI507, 1717-2A>G, R1283M, R117C, 3617G/T, 621+2T>C or F508C alleles were present. These data demonstrate that, for the clinical material available to us, one allele does not fail to amplify when combined with another in the same ARMS tube and that one allele does not give rise to artefactual products derived from alternative allele-specific primers. Table 3 provides an analysis of the 754 chromosomes typed using CF(12)m ARMS test.

Early diagnosis followed by expert management has resulted in an improved prognosis for CF patients. This has led to the evaluation of neonatal screening protocols for CF. There is also a common objective of identifying carriers and ultimately those couples who are at risk of having a child affected with CF that is shared by 'Active cascade'¹², 'Couple'^{13,14} and 'Two step' or 'Stepwise'^{14,15} screening protocols. It has also been highlighted that there are risks associated with *in vitro* fertilisation. Up to ten offspring may be fathered by one sperm donor. If a donor is a CF carrier there will be a higher than normal chance of at least one of the offspring being affected by the disease. For this reason it was recommended that sperm donors should be routinely tested for mutations in the CFTR gene¹⁶. The primary application of the CF(12)m test is screening individuals who may be carriers of one of the CF mutations 1717-1G>A, G542X, W1282X, N1303K, AF508, 3849+10kb C>T, 621+1 G>T, R553X, G551D, R117H, R1162X and R334W, the most common CF mutations in Caucasians and Ashkenazi Jews. In Europe, CF individuals who are non-AF508 CF compound heterozygotes are rare. As a consequence it has not been possible to analyse for all mutations in combination in the same test. However, nine compound heterozygotes have been sourced and analysed. In each of these cases and in all AF508 CF compound heterozygotes both mutations were correctly identified clearly demonstrating the diagnostic value of the test.

There is a clear difficulty in distinguishing particular alleles in the routine genetic screening laboratory¹⁷. Cuppens and Cassiman demonstrated that 12.5% of laboratories mistyped the F508C polymorphism as a true mutation and that 12.5% confused the ΔI507 mutation for ΔF508¹⁷. One conclusion of that study was that the accuracy of CFTR typing should be improved¹⁷. The CF(12)m test was designed such that a false result does not arise because of other DNA sequence variations in the vicinity of the mutations tested for. This was achieved through the design and choice of transcriptional orientation of each respective ARMS primer. The CF(12)m test accurately discriminates between ΔF508 and non-ΔF508 alleles which are in turn distinguished from the mutant ΔI507 and the benign F508C alleles.This addresses the the problems associated with typing particular alleles identified by Cuppens and Cassiman. Furthermore, findings from another study¹⁴, that compared multiplex ARMS screening for the ΔF508, G551D, G542X and 621+1 G>T alleles⁸ with alternative routine procedures for the same alleles were that multiplex ARMS was the prefered method.

Standardisation of all test procedures and implementation of appropriate quality control measures can control for some test variables and it is important to understand which ones are likely to affect test performance. The annealing temperature is one key parameter which can affect the performance of any PCR or ARMS reaction. If the reaction temperature falls below the required annealing temperature it is likely that reaction specificity will be affected. Similarly, if the annealing temperature is higher than that required the efficiency of the ARMS reaction may be reduced resulting in PCR failure. There is expected to be some degree of variability between the temperature accuracy and uniformity exhibited by different thermal cyclers. We therefore established the temperature range within which all the component reactions of the test would function (at least +/-2 °C). All thermal cyclers currently available commercially are claimed by the requisite manufacturer to have a temperature accuracy of +/-1 °C or better. We have similarly determined the range within which other parameters can vary without adversely affecting test function. In defining these we have established the tolerances within which the kit must be assembled. The two key variables that can be influenced by the kit user are the annealing temperature of the ARMS reaction and the amount of the *Taq* DNA polymerase added to individual reaction mixes. We have addressed the function of the test when the upper and lower limits of these variables are applied in concert. From these studies, even with low enzyme and high annealing temperature and vice versa, the control amplicons are produced and the test does not give false results using our panel of DNAs. We therefore conclude that the CF(12)m test is reliable and robust and is unaffected by external variations so long as the kit instructions and recommendations are adhered to. If a reaction should fail for any other reason that is not addressed by the parameters investigated herein, a misdiagnosis would still not occur since a control amplicon would be eliminated prior to any ARMS amplicon. This feature emphasises the importance of the internal controls and their role in avoiding misdiagnoses. There were six archival DNA samples that failed to produce one or both control bands, these may have been degraded and they were not scored. Because the CF(12)m test is primarily a screening test, by implication DNA used in the test will be freshly prepared. The age and degradation of a sample is therefore not believed to be a problem during the test's routine application.

### Example 2

### Clinical evaluation of the CF(12)m cystic fibrosis DNA diagnostic kit

Forty DNA samples from thirty nine previously genotyped cystic fibrosis (CF) patients or carriers and one unaffected individual were evaluated independently in a blind study by two genetic testing laboratories involved in CF molecular diagnosis performing 500 diagnoses per year combined. The CF(12)m kit uses multiplexed amplification refractory mutation system (ARMS™) technology which allows the simultaneous identification of the more prevalent CFTR gene mutations⁵ 1717-1G>A, G542X, W1282X, N1303K, ΔF508, 3849+10kb C>T, 621+1 G>T, R553X, G551D, R117H, R1162X and R334W in one working day. The kit was found to be accurate and reliable.

### Methods

### Samples

Forty DNA samples from CF patients or carriers and an unaffected individual were typed. The reference method for DNA typing was the analysis of the 27 exons and the intronexon boundaries of the CFTR gene as described previously⁶. Six further samples bearing the ΔI507, R117C, R347H, D1152H and R117P mutations and one bearing the 1540A->G polymorphism, not detectable with the CF(12)m kit, were also tested to evaluate cross reactivity. Whole blood had been stored at -20_C prior to CF(12)m kit analysis.

### DNA analysis using CF(12)m kits

DNA was extracted from thawed blood and analysed. After DNA amplification the reaction mixtures were separated by agarose gel electrophoresis to reveal the diagnostic and amplification control DNA fragments³.

### Results

The extraction often blood samples could be completed in half a day. DNA was successfully amplified from all samples, however, one remained dark brown after washing and could not be amplified initially. This DNA was diluted ten fold after which it amplified successfully.

All samples were analysed in both laboratories. In one lab one sample required a repeat analysis because of the absence of a control DNA fragment. One sample, different in each lab (see Table 1), was not interpreted even after reamplification. In lab 1 sample six had an allele carrying the 1717-1G->A mutation which was amplified only at a low level. In lab 2 sample 26 showed no amplification control band and no mutant ARMS products.

As expected, the mutations 2789+5 G->A, 2176insC, 1078delT and S1235R and the polymorphisms 875+40A/G and 2694A/C identified by the reference method were not detected by the kit, neither were they erroneously typed. This was also found for the ΔI507, R117C, R347H, D1152H, R117P 1540A->G samples [data not shown]. None of the CF mutations detectable by the kit were incorrectly identified as other mutations.

In all cases with the exception of samples six and 26 where one or more of the 12 mutations detected by the kit were present, the kit accurately identified them. When compared to the reference method the kit detected the same mutations in 34 of the 40 samples (85%), (see Table 1). Four samples carried a rarer mutation that is not detected by the kit. Two other samples were from patients homozygous for the W1282X and G542X mutations. Figure 1 shows a result typical of those produced using the CF(12)m kit.

The CF(12)m kit, uses multiplex ARMS technology which is extremely rapid and allows the screening of at least ten samples for 12 CFTR mutations in a working day. A major feature of the kit therefore is the speed with which the results are delivered. This allows the laboratory staff to undertake additional activities and it increases sample throughput. We found the kit to be reliable and accurate after evaluating it in a blind study in two independent laboratories both of which are experienced in CF molecular diagnostics. The results obtained were in accordance with those described by the manufacturer and demonstrated a first time amplification success rate of 97%.

In one laboratory, the absence or low level of the amplification control DNA fragment was observed and this was improved by the analysis of small batches of samples. This probably reflects reduced denaturation of the DNA polymerase during the hot-start prior to thermal cycling. To achieve consistently reliable results we found that strict adherence to the protocols was required. The control DNA fragments should be visible routinely to avoid the risk of getting a false negative result. The absence of the upper control fragment in conjunction with no mutant allele amplicons indicate that a repeat test is required.

The 12 mutations that the CF(12)m kit can detect are among the most frequent observed in European populations. The diagnostic power of the test, depending on the population under study has been calculated. In the French population generally, 78.8% of CF chromosomes should be detected using the CF(12)m kit, in our hands 85% were detected in both laboratories. This seems satisfactory as screening for very rare mutations does not significantly increase the detection frequency of CF chromosomes.

A potential limitation of the CF(12)m kit is that it does not distinguish between homozygotes and heterozygotes for mutations other than AF508. Although homozygotes for the rarer mutations are usually encountered infrequently, this characteristic of the kit must be considered when interpreting the test result. This was demonstrated in this investigation when the genotype of the W1282X and G542X homozygous patients could not be determined since the kit does not differentiate between one or two mutated alleles except for the AF508 mutation.

The identification of mutations in the CFTR gene is required primarily in patients suspected of suffering from CF and is important for genetic counselling in the patient family. The CF(12)m kit is useful in this case for first line screening and can be complemented by other methods if two mutations are not detected initially. The detection rate of the kit allows the calculation by Bayesian analysis of the posterior risk of an 'at risk individual', with a negative result. Similar assessments of carrier risk estimation can be calculated for individuals with a family history of CF where affected or obligate heterozygous individuals are not available for mutation typing.

The CF(12)m kit will also be useful in CF carrier screening programs and of particular use in CF neonatal screening in association with immunoreactive trypsin (IRT) (Wilcken et al, J.Pediat., 1995, 127, 965-970) or pancreatitis-associated protein (PAP) (CR.Acad.Sci.Paris/Life Sciences, 1994, 317, 561-564).

**Table 1**

| **Country** | **CF chromosomes (%)** |
|---|---|
| Albania | 75.0 |
| Austria | 72.2 |
| Belgium | 84.5 |
| Bulgaria | 67.1 |
| Czech Republic | 80.6 |
| Denmark | 91.0 |
| France | 78.8 |
| Germany | 78.6 |
| Hungary | 73.0 |
| Greece | 68.7 |
| Ireland | 79.1 |
| Italy | 60.3 |
| Netherlands | 83.5 |
| Poland | 65.0 |
| Portugal | 48.4 |
| Russia | 53.4 |
| Slovenia | 69.1 |
| Spain | 63.2 |
| Sweden | 56.0 |
| Switzerland | 80.3 |
| United Kingdom | 81.3 |

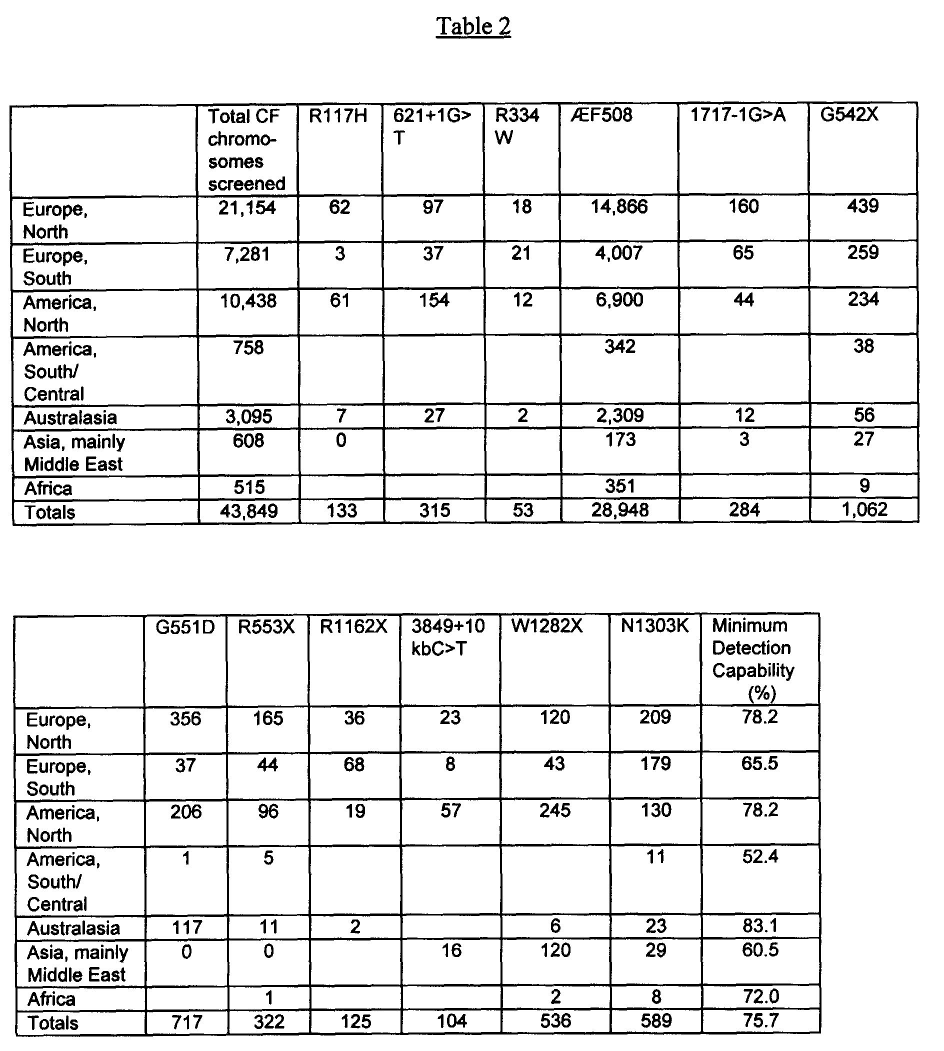

**Table 3**

| Mutation | Independent typing method^{a} | Totals |
|---|---|---|
| 1717-1G>A | ASO | 16 |
| G542X | ASO | 10 |
| W1282X | ASO | 16 |
| N1303K | ASO | 12 |
| *A*EF508 | Electrophoresis | 89 |
| 3849+10kb C>T | Digest (Hphl) | 11 |
| 621+1 G>T | Digest (Msel) | 7 |
| R553X | Digest (Hincll) | 15 |
| G551D | Digest (Ndel) | 16 |
| RII7H | ASO | 13 |
| R1162X | Digest (Ddel) | 11 |
| R334W | Digest (Mspl) | 6 |
| Other/none | | 532 |
| | | |
| Number of samples | | 377 |
| Total number of chromosomes | | 754 |

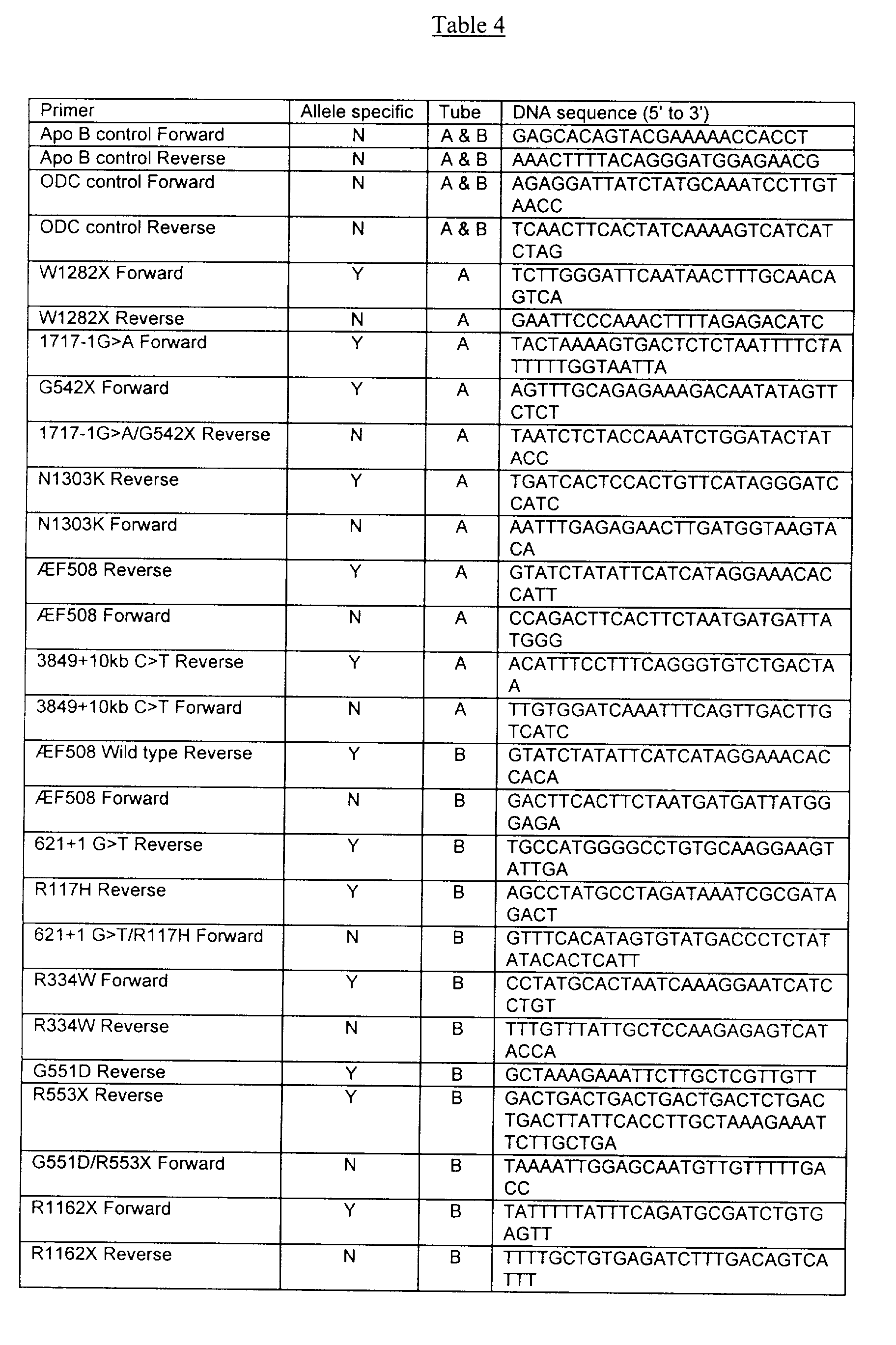

## Claims

1. A method for detecting the presence or absence of twelve mutations in the cystic fibrosis transmembrane conductor regulator (CFTR) gene, which method comprises contacting sample genomic DNA from an individual in two separate reaction vessels with allele specific primer sets for (A) 1717-1 G>A, G542X, W1282X, N1303K, ΔF508(M), 3849+10kb C>T mutations and (B) the 621+1 G>T, R553X, G551D, R117H, R1162X and R334W mutations respectively, in the presence of appropriate nucleotide triphosphates and an agent for polymerisation, such that each diagnostic primer is extended only when the relevant mutation is present in the sample; and detecting the presence or absence of CFTR gene alleles by reference to the presence or absence of diagnostic primer extension product(s).

2. A method as claimed in claim 1 and wherein one or more diagnostic primers is used with one or more amplification primers in one or more cycles of PCR amplification.

3. A set of allele specific primers for each of the following alleles of the CFTR gene: 1717-1 G>A, G542X, W1282X, N1303K, ΔF508(M), and 3849+10kb C>T mutations.

4. A set of primers as claimed in claim 3 and comprising the following diagnostic primer sequences:

TCTTGGGATTCAATAACTTTGCAACAGTCA
TACTAAAAGTGACTCTCTAATTTTCTATTTTTGGTAATTA
AGTTTGCAGAGAAAGACAATATAGTTCTCT
TGATCACTCCACTGTTCATAGGGATCCATC
GTATCTATATTCATCATAGGAAACACCATT
ACATTTCCTTTCAGGGTGTCTGACTAA

5. A set of allele specific primers for each of the following alleles of the CFTR gene: 621+1 G>T, R553X, G551D, R117H, R1162X and R334W mutations.

6. A set of primers as claimed in claim 5 and comprising the following diagnostic primer sequences:

GTATCTATATTCATCATAGGAAACACCACA
TGCCATGGGGCCTGTGCAAGGAAGTATTGA
AGCCTATGCCTAGATAAATCGCGATAGACT
CCTATGCACTAATCAAAGGAATCATCCTGT
GCTAAAGAAATTCTTGCTCGTTGTT
GACTGACTGACTGACTGACTCTGACTGACTTATTCA
CCTTGCTAAAGAAATTCTTGCTGA
TATTTTTATTTCAGATGCGATCTGTGAGTT

7. A set of primers comprising the following diagnostic primer and amplification primer sequences:

TCTTGGGATTCAATAACTTTGCAACAGTCA
GAATTCCCAAACTTTTAGAGACATC
TACTAAAAGTGACTCTCTAATTTTCTATTTTTGGTAATTA
AGTTTGCAGAGAAAGACAATATAGTTCTCT
TAATCTCTACCAAATCTGGATACTATACC
TGATCACTCCACTGTTCATAGGGATCCATC
AATTTGAGAGAACTTGATGGTAAGTACA
GTATCTATATTCATCATAGGAAACACCATT
CCAGACTTCACTTCTAATGATGATTATGGG
ACATTTCCTTTCAGGGTGTCTGACTAA
TTGTGGATCAAATTTCAGTTGACTTGTCATC

8. A set of primers comprising the following diagnostic primer and amplification primer sequences:

GTATCTATATTCATCATAGGAAACACCACA
GACTTCACTTCTAATGATGATTATGGGAGA
TGCCATGGGGCCTGTGCAAGGAAGTATTGA
AGCCTATGCCTAGATAAATCGCGATAGACT
GTTTCACATAGTGTATGACCCTCTATATACACTCATT
CCTATGCACTAATCAAAGGAATCATCCTGT
TTTGTTTATTGCTCCAAGAGAGTCATACCA
GCTAAAGAAATTCTTGCTCGTTGTT
GACTGACTGACTGACTGACTCTGACTGACTTATTCA

CCTTGCTAAAGAAATTCTTGCTGA
TAAAATTGGAGCAATGTTGTTTTTGACC
TATTTTTATTTCAGATGCGATCTGTGAGTT
TTTTGCTGTGAGATCTTTGACAGTCATTT

9. A set of primers as claimed in any one of the previous claims and comprising one or more of the following control primers:

GAGCACAGTACGAAAAACCACCT
AAACTTTTACAGGGATGGAGAACG
AGAGGATTATCTATGCAAATCCTTGTAACC
TCAACTTCACTATCAAAAGTCATCATCTAG

10. A diagnostic kit for detecting the presence or absence of twelve mutations in the cystic fibrosis transmembrane conductor regulator (CFTR) gene which comprises sets of primers as claimed in any of the previous claims.
